# EUROPÄISCHE PATENTSCHRIFT

(11) **EP 3 248 683 B1**
(45) Veröffentlichungstag und Bekanntmachung des Hinweises auf die Patenterteilung: **07.04.2021**
(21) Anmeldenummer: 17168980.5
(22) Anmeldetag: 02.05.2017
(51) Int. Cl.: B01L 3/02, A61B 5/15, B01L 9/06

(54) **KAPILLARENTLEERUNGSHILFE**
CAPILLARY DRAINING AID
AIDE À L'ÉCOULEMENT CAPILLAIRE

(30) Priorität: 24.05.2016 DE 102016109536
(43) Veröffentlichungstag der Anmeldung: 29.11.2017
(73) Patentinhaber: KABE-Labortechnik GmbH, 51588 Nümbrecht-Elsenroth (DE)
(72) Erfinder: Kötter, Frank, 51789 Lindlar (DE)
(74) Vertreter: Christophersen & Partner Partnerschaft mbB Patentanwälte

(56) Entgegenhaltungen:
- EP-A1- 2 031 408
- EP-A2- 0 232 517
- WO-A1-2011/075075
- WO-A2-99/51350
- DE-A1- 3 701 250
- DE-U1-202004 004 951

## Beschreibung

Im Allgemeinen betrifft die Erfindung eine Pipettiervorrichtung zur Aufnahme und Abgabe von medizinischen Proben, zum Beispiel Körperfluiden, mit einem zylindrischen Kapillarröhrchen und einem Röhrchenhalter, wobei der Rörchenhalter besteht aus
- einem Kanalabschnitt zur Aufnahme des Kapillarröhrchens,
- einem radial erweiterten Abschnitt, der dem Kanalabschnitt abgewandt mit einer Öffnung versehen ist,
- einem Verbindungsabschnitt, welcher eine Strömungsverbindung zwischen dem Kanalabschnitt und dem radial erweiterten Abschnitt bildet,
wobei das gemeinsame Volumen des Verbindungsabschnitts und des radial erweiterten Abschnitts ein Mehrfaches des von dem Kapillarröhrchen umschlossenen Kapillarvolumens beträgt und verkleinerbar ist.

Aus der WO 2011/075075 ist eine Pipettiervorrichtung zur Flüssigkeitsaufnahme von bis zu 200 µL bekannt. Die dort gezeigte Mikropipette setzt sich aus einem Kapillarröhrchen und einem das Kapillarröhrchen an einem Ende aufnehmenden Rohrabschnitt zusammen. Der Rohrabschnitt schließt an seinem anderen Ende mit einer Gasaustauscheinheit ab. Diese wird zu ihrer Befestigung über einen an dem Rohrabschnitt angeformten Flansch gestülpt. Ein an dem Rohrabschnitt ausgebildeter Flansch dient der Handhabung der Pipettiervorrichtung. Zum Ansaugen von Flüssigkeit wird zunächst die innerhalb der Austauscheinheit befindliche Luft herausgedrückt und die Flüssigkeit durch Unterdruck oder unter Kapillarzug in das Kapillarröhrchen aufgesogen. Zur späteren Abgabe des Fluids wird die Gasaustauscheinheit durch Druck mit dem Finger betätigt. Um ein Eindringen von Wasser, Blut oder Körperfluiden in den Rohrabschnitt zu vermeiden, ist dieser aus einem weniger hydrophilen Material gefertigt als das Kapillarröhrchen.

Von Nachteil ist, dass zunächst die aus Silikon oder Gummi bestehende Gasaustauscheinheit vom Anwender auf den Rohrabschnitt gestülpt werden muss. Für dieses Überstülpen ist zwar ein gewisser Ausgleich des in der Gasaustauscheinheit eingeschlossenen Luftvolumens vorgesehen, jedoch kann es, wenn sich im Kapillarröhrchen bereits eine Flüssigkeitsprobe befindet, doch zu einem teilweisen Austreten und damit einem vorzeitigen Flüssigkeitsverlust kommen.

Außerdem ist das Aufstülpen der Gasaustauscheinheit ein zusätzlicher Verfahrensschritt. Weiterhin bedarf diese Konstruktion mehrerer Schritte zur Fertigung der Einzelkomponenten. Schließlich kann die Gefahr einer Kontamination der Gasaustauscheinheit mit angesaugtem Fluid nicht ausgeschlossen werden, was insbesondere bei Mehrfachverwendung der Gasaustauscheinheit problematisch sein kann.

Aus der DE 10 2004 021 741 A1 und der WO 2005/094681 A1 sind Pipettiervorrichtungen bekannt, bei welchen ein Kapillarröhrchen oder eine Kanüle mit einem seiner Enden in eine Öffnung eines langgestreckten Röhrchenhalters eingesetzt wird. In dessen Hauptkörper ist längsbeweglich ein Kolben angeordnet. Dabei kann der Kolben eine Öffnung zur Entlüftung des Hauptkörpers besitzen, oder er ist leicht beabstandet zur Innenwand des Hauptkörpers angeordnet. Zum Aufnehmen von zum Beispiel Blut ist der Kolben nahezu vollständig aus dem den Kolben führenden Zylinderabschnitt des Hauptkörpers herausgezogen. Die Mikropipette bzw. die Kapillare oder Kanüle wird mit ihrem offenen Ende solange in Kontakt zum Fluid gebracht, bis durch Kapillarzug das gewünschte Fülllevel im Kapillarröhrchen erreicht ist. Durch Herunterdrücken des Kolbens, bei durch einen Finger verschlossener Öffnung, wird das Kapillarröhrchen entleert. Bei Vorrichtungen dieser Art ist es von Nachteil, dass der Kolben als separates Bauteil gefertigt ist, und somit zunächst montiert werden muss

Auch EP-A-0 232 517, WO 99/51350, EP-A-2 031 408 und DE 20 2004 004951 U beschreiben Pipettiervorrichtungen.

Der Erfindung liegt die Aufgabe zugrunde, eine mit geringem Aufwand herstellbare Pipettiervorrichtung der eingangs genannten Art bereitzustellen, welche die Handhabung erleichtert und die Sicherheit für den Anwender erhöht.

Zur Lösung der Aufgabe wird eine Pipettiervorrichtung mit den Merkmalen des Anspruchs 1 vorgeschlagen.

Vorteilhafte Ausgestaltungen der Pipettiervorrichtung sind in den Unteransprüchen angegeben.

Die erfindungsgemäße Pipettiervorrichtung ist zur Aufnahme und Abgabe von medizinischen Proben, insbesondere Körperfluiden wie z. B. Blut, geeignet. Auch können andere Flüssigkeiten, Dispersionen, Emulsionen oder gar aufgewirbelte Suspensionen mit einer solchen Pipettiervorrichtung aufgenommen werden. Die Probenauswahl ist nicht auf medizinische Proben beschränkt.

Die Pipettiervorrichtung zur Aufnahme und Abgabe von medizinischen Proben, zum Beispiel Körperfluiden, umfasst ein zylindrisches Kapillarröhrchen und einen Röhrchenhalter. Dieser besteht aus
- einem Kanalabschnitt zur Aufnahme des Kapillarröhrchens,
- einem radial erweiterten Abschnitt, der dem Kanalabschnitt abgewandt mit einer Öffnung versehen ist,
- einem Verbindungsabschnitt, welcher eine Strömungsverbindung zwischen dem Kanalabschnitt und dem radial erweiterten Abschnitt bildet,
wobei das gemeinsame Volumen des Verbindungsabschnitts und des radial erweiterten Abschnitts ein Mehrfaches des von dem Kapillarröhrchen umschlossenen Kapillarvolumens beträgt, und dieses gemeinsame Volumen verkleinerbar ist.

Hierbei ist der Röhrchenhalter zur Verkleinerung des gemeinsamen Volumens mit einem in Längsrichtung des Röhrchenhalters verformbar ausgebildeten Stauchabschnitt, der einstückig mit dem Verbindungsabschnitt und mit dem radial erweiterten Abschnitt ist, versehen. Vorzugsweise ist der Stauchabschnitt reversibel verformbar ausgebildet, d. h. er nimmt nach der Verformung wieder seine ursprüngliche Form an.

Außerdem ist der Kanalabschnitt einstückig mit dem Verbindungsabschnitt ausgebildet. Dies erhöht die Stabilität der Gesamtkonstruktion, vereinfacht die Fertigung und gewährleistet eine sichere Aufnahme des Kapillarröhrchens in dem Kanalabschnitt des Röhrchenhalters.

Der Röhrchenhalter kann, einschließlich des radial erweiterten Abschnitts und des Stauchabschnitts, in einem einzigen Schritt als ein einstückiges, d. h. materialeinheitliches Teil hergestellt werden, z. B. in einem Spritzgussverfahren. Dies ist insbesondere für die preiswerte Massenproduktion der Einweg-Pipettiervorrichtung günstig. Auch wird die Handhabung der Pipettiervorrichtung erleichtert, da ein separates Aufstülpen einer Gasaustauscheinheit durch den Anwender, wie bei der aus der WO 2011/075075 bekannten Mikropipette, entfällt. Darüber hinaus steigt auch die Zuverlässigkeit der Pipettiervorrichtung, da kein Aufsetzen einer Gasaustauscheinheit gemäß der WO 2011/075075 oder die Montage eines Kolbens gemäß der DE 10 2004 021 741 A1 oder der WO 2005/094681 A1 notwendig ist. Die Gefahr eines unsachgemäßen Zusammenbaus oder gar einer Beschädigung bei der Montage entfällt.

Zwischen dem Kanalabschnitt und dem Verbindungsabschnitt ist eine Verengung ausgebildet. Diese dient einerseits der Materialstabilisierung, dient gleichzeitig aber auch als Abdichtung des Innenraums des Verbindungsabschnitts gegen übertretendes oder spritzendes Fluid aus dem Kapillarröhrchen beziehungsweise dem Kanalabschnitt. Weiterhin kann die Verengung auch Befestigungsflansch für Filter wie beispielsweise einen Fluid- oder Blutstopp sein.

Ein solcher Fluidstopp kann entweder auf der dem Kapillarröhrchen zugewandten Seite oder auf der dem Kapillarröhrchen abgewandten Seite oder in der Verengung angeordnet sein. Die Verengung dient auch als Auflage- oder Befestigungsflansch für den Fluidstopp.

Erfindungsgemäß ist ein für Luft oder Inertgas durchlässiger, aber für Flüssigkeiten und Feststoffe weitgehend undurchlässiger Fluidstopp im Bereich der Verengung versehen. Dadurch wird die Gefahr verringert, dass möglicherweise viral, bakteriell oder anders kontaminierte Fluide in den Verbindungsabschnitt eindringen und möglicherweise durch den einseitig offenen radial erweiterten Abschnitt nach außen dringen und gar den Anwender kontaminieren.

In einer bevorzugten Ausgestaltung ist an dem Verbindungsabschnitt des Röhrchenhalters ein Flansch starr und einstückig angeformt. Dies ermöglicht dem Anwender ein sicheres Greifen und Halten der Pipettiervorrichtung. Außerdem bildet der Flansch ein Widerlager, an dem sich die Finger des Benutzers beim Entleeren der Pipettiervorrichtung abstützen können.

In einer weiteren Ausgestaltung ist der Fluidstopp derart ausgebildet, dass er sich durch den Kontakt mit Feuchtigkeit verdichtet. So kann die ohnehin in den Proben auftretende Feuchtigkeit bei Kontakt mit dem Fluidstopp in-situ ausgenutzt werden. Dies erhöht die Sicherheit bei der Handhabung und schützt den Anwender vor Kontamination mit der Probe.

In einer weiteren Ausgestaltung ist die Innenseite des Kanalabschnittes zu dessen offenem Ende hin konisch erweitert ausgebildet. Durch den konisch erweiterten Endbereich wird die Aufnahme des Kapillarröhrchens erleichtert, welches von geringerem Außendurchmesser als der Durchmesser der Innenwandung des konisch erweiterten Endes ist. Selbst wenn nur ein kurzer Abschnitt des Kapillarröhrchens bei dessen Aufnahme erfasst wird, gleitet dieser längs der konischen Öffnung in den Kanalabschnitt hinein, und kann dann anschließend durch Klemmen gehalten werden.

Trotz des innen konisch verlaufenden Kanalabschnitts, verjüngt sich der Röhrchenhalter von außen in Richtung des Kapillarröhrchens. Dabei verjüngen sich sowohl der Verbindungsabschnitt als auch der Kanalabschnitt, wodurch eine hohe Stabilität des Röhrchenhalters sichergestellt ist.

Vorzugsweise ist der durch Stauchen verformbare Abschnitt von Ziehharmonika-förmiger Gestalt. Dabei setzt er sich einstückig aus zickzackförmig einander abwechselnden Wandabschnitten zusammen. Bevorzugt nimmt der Durchmesser der zickzackförmigen Wandabschnitte in der dem Kapillarröhrchen abgewandten Richtung zu. Der Vorteil dieser Anordnung ist die Möglichkeit der einstückigen Fertigung und des zuverlässigen, vorzugsweise reversiblen Stauchens beim Zusammenschieben der Falten. Die einstückige Fertigung erspart Materialkosten und verbessert für den Anwender die Nutzung.

Vorzugsweise ist der Röhrchenhalter samt Kanalabschnitt, Verbindungsabschnitt, dem radial erweiterten Abschnitt und dem Stauchabschnitt ein Spritzgussteil aus einem polyolefinischen Material. Insbesondere bevorzugt ist dabei Polyethylen (PE), beschränkt sich aber nicht auf dessen Einsatz. Das Spritzgussverfahren ermöglicht eine kostengünstige und schnelle Massenfertigung. Aus den Materialeigenschaften von Polyethylen ergibt sich für den Röhrchenhalter samt den genannten Abschnitten ein relativ geringes Gewicht, eine Ermüdungsbeständigkeit sowie eine chemische Inertheit gegenüber den meisten organischen Lösungsmitteln.

Bevorzugt ist es, das Kapillarröhrchen aus transparentem Polyvinylchlorid (PVC) oder Polyethylenterephthalat (PET) zu fertigen. Dieses ist chemisch relativ inert und widerstandsfähig. Die Verarbeitung erfolgt durch Extrusion, wodurch die Kapillarröhrchen kostengünstig und in hoher Stückzahl herstellbar sind. Die Kapillarröhrchen können auch aus anderen Materialien bestehen, beispielsweise Glas.

Eine Ausführungsform der erfindungsgemäßen Pipettiervorrichtung wird im Folgenden anhand der Zeichnungen näher erläutert. Es zeigen:
- Fig. 1: eine Schnittdarstellung der Pipettiervorrichtung während der Aufnahme des Kapillarröhrchens aus einem Aufbewahrungsbehältnis, samt einer Vergrößerung;
- Fig. 2: eine Schnittdarstellung der Pipettiervorrichtung mit im Behältnis des Kapillarröhrchens aufgenommenem Kapillarröhrchen;
- Fig. 3: eine Schnittdarstellung der Pipettiervorrichtung nach der Aufnahme des Kapillarröhrchens aus dem Behältnis;
- Fig. 4: schematische Darstellung der Aufnahme eines Bluttropfens mit der Pipettiervorrichtung;
- Fig. 5: schematische Darstellung der Pipettiervorrichtung kurz vor dem Entleeren des aufgenommenen Fluids in ein Probenbehältnis;
- Fig. 6: schematische Darstellung der Pipettiervorrichtung beim Entleeren des aufgenommenen Fluids in ein Probenbehältnis;
- Fig. 7: schematische Darstellung der Pipettiervorrichtung bei der teilweisen Abgabe des aufgenommenen Fluids auf einen Laborteststreifen.

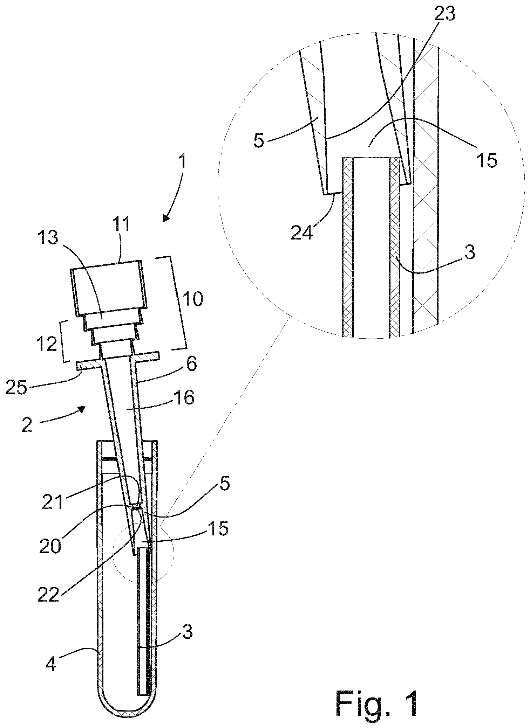

Die erfindungsgemäße Pipettiervorrichtung 1 zur Aufnahme von medizinischen Proben, zum Beispiel Körperfluiden und insbesondere Blut, setzt sich aus einem Röhrchenhalter 2 und einem innen und außen zylindrischen Kapillarröhrchen 3 zusammen. Wie aus Figur 1 ersichtlich, sind der Röhrchenhalter 2 einerseits und das Kapillarröhrchen 3 andererseits separate Bauteile, sie werden also unabhängig voneinander gefertigt.

Das Kapillarröhrchen 3 kann sich vor der Probenaufnahme oder der Probenabgabe in einem Aufbewahrungsbehältnis 4 befinden, etwa einem Reagenzglas oder einem Probenröhrchen. Selbstverständlich können darin auch mehrere derartige Kapillarröhrchen 3 vorhanden sein.

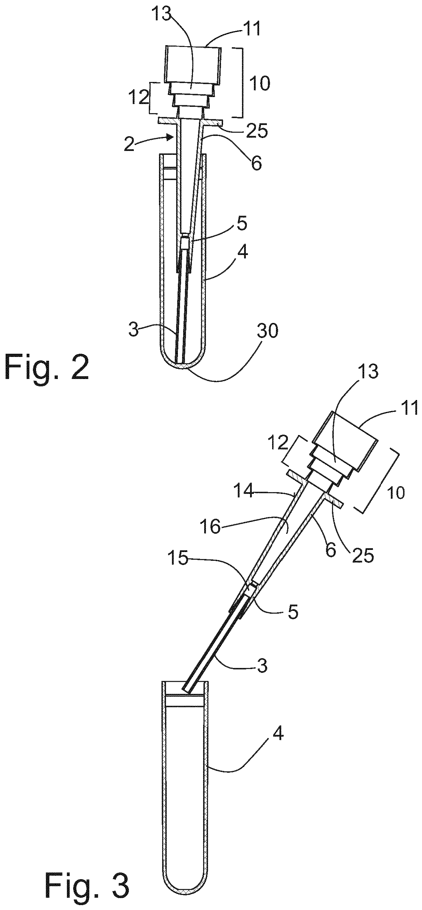

Zur Aufnahme und zur Befestigung des Kapillarröhrchens 3 am Röhrchenhalter 2 wird dieser mit seiner Öffnung gegen das eine Ende des Kapillarröhrchens 3 gedrückt. Dabei dient eine konisch gestaltete Einlaufschräge innen am Kanalabschnitt 5 als wichtige Hilfe zum Aufnehmen des Kapillarröhrchens 3 aus dem Aufbewahrungsbehältnis 4. Wie in Figur 2 gezeigt, hat der Boden 30 des Aufbewahrungsbehältnisses 4 dabei die Funktion des Widerlagers. Sobald durch axialen Druck gegen das so abgestützte Kapillarröhrchen 3 ein leichter Presssitz des Kapillarröhrchens im Röhrchenhalter 2 erreicht ist, kann die Pipettiervorrichtung 1 verwendet werden.

Der Röhrchenhalter 2 setzt sich aus mehreren hintereinander angeordneten und miteinander in Strömungsverbindung stehenden Abschnitten zusammen, nämlich einem Kanalabschnitt 5, einem Verbindungsabschnitt 6 und einem radial erweiterten Abschnitt 10. Strömungsverbindung meint hier die Strömungsmöglichkeit des Fluids beim Ansaugen oder Entleeren in bzw. aus der Pipettiervorrichtung 1.

Der Röhrchenhalter 2 ist, dem Kapillarröhrchen 3 zugewandt, durch einen zum Kapillarröhrchen 3 hin offenen Kanalabschnitt 5 abgeschlossen. Der Kanalabschnitt 5 dient zur Aufnahme und Befestigung des Kapillarröhrchens 3. An den Kanalabschnitt 5 schließt sich einstückig der Verbindungsabschnitt 6 an, der die Strömungsverbindung zwischen dem Kanalabschnitt 5 und dem radial erweiterten Abschnitt 10 bildet. Der Verbindungsabschnitt 6 ist einstückig mit einem radial erweiterten Abschnitt 10. Der radial erweiterte Abschnitt 10 weist ein im Vergleich zu dem Inneren des Kapillarröhrchens vielfach größeres Volumen auf. Er ist dem Kapillarröhrchen 3 bzw. dem Kanalabschnitt 5 abgewandt mit einer Öffnung 11 versehen, deren Öffnungsquerschnitt zum Beispiel gleich ist dem Querschnitt des radial erweiterten Abschnitts 10.

Bestandteil des radial erweiterten Abschnitts 10 ist ein reversibel verformbarer, Ziehharmonika-artig geformter Stauchabschnitt 12. Der Stauchabschnitt 12 besteht aus zickzackförmig aneinandergereihten Segmenten 13 unterschiedlichen Durchmessers quer zur Längsachse der Pipettiervorrichtung 1. Der Stauchabschnitt 12 ist einstückig sowohl mit dem Verbindungsabschnitt 6 als auch mit dem radial erweiterten Abschnitt 10.

Der Stauchabschnitt 12 kann aus einem ursprünglich relaxierten Zustand in einen in Längsrichtung komprimierten Zustand verformt werden. Dabei reduziert sich das Luftvolumen in dem Abschnitt 10, so dass Luft aus dem Röhrchenhalter 2 zu dem Kapillarröhrchen 3 hin strömt, und das dort vorhandene Fluid verdrängt. Dabei beträgt das gemeinsame Volumen des Verbindungsabschnitts 6 und des radial erweiterten Abschnitts 10 ein Mehrfaches des von dem Kapillarröhrchen 3 umschlossenen Kapillarvolumens.

Die Betätigung des Stauchabschnitts 12 wird über Druck mit einem Finger oder dem Daumen vollzogen, wobei zugleich die Öffnung 11 durch den Finger bzw. Daumen verschlossen ist. Nach der Kompression dehnt sich der Stauchabschnitt 12 aufgrund der Materialelastizität wieder aus und gelangt reversibel zurück in seine Ausgangslage. Dies kann je nach Material variieren. Für einen Einweg-Gebrauch allerdings ist eine reversible Verformbarkeit nicht zwingend.

Wie unter anderem Figur 1 zeigt, verjüngen sich der Kanalabschnitt 5 und der Verbindungsabschnitt 6 zu dem Kapillarröhrchen 3 hin. Die Mantelform innen im Bereich des Kanalabschnitts 5 und des Verbindungsabschnitts 6 ist kegelartig.

Der durch den Kanalabschnitt 5 und den Verbindungsabschnitt 6 sich erstreckende Innenraum 15, 16 ist an der Verbindungsstelle zwischen Kanalabschnitt 5 und Verbindungsstelle 6 mit einer Verengung 20 versehen. Die Verengung 20 ist ein einstückig angeformter, radial nach innen weisender Bund oder Flansch 20, der jedoch in seiner Mitte einen Strömungsdurchgang freilässt.

Im Bereich der zentralen Öffnung 21 der Verengung 20 ist ein Fluidstopp 22 eingesetzt oder befestigt. Der Fluidstopp 22 zeichnet sich dadurch aus, dass er sich durch den Kontakt mit Feuchtigkeit verdichtet. Der Fluidkontakt mit dem Fluidstopp 22 kann beispielsweise durch vertikal nach oben spritzendes Fluid aus dem Kapillarröhrchen 3 bedingt sein, etwa bei einem Ansaugprozess. Während der Fluidstopp 22 weitgehend undurchlässig für Flüssigkeiten ist, ist er durchlässig für den Gasaustausch. Dies ist beispielsweise notwendig, um das Kapillarröhrchen 3 unter Gasdruck zu entleeren, bewerkstelligt durch Druck auf den Stauchabschnitt 12 bei verschlossener Öffnung 11.

Wie die Vergrößerung in Fig. 1 zu erkennen gibt, ist die Innenwandung 23 des Kanalabschnittes 5 zu dessen offenem Ende 24 hin konisch erweitert ausgebildet. Der Innendurchmesser des Kanalabschnitts 5 ist also im Bereich der Verengung 20 kleiner als im Bereich der Öffnung 24. Dadurch, dass sich der Innendurchmesser zu dem Kapillarröhrchen 3 hin erweitert, ähnlich einem Trichter, und er insbesondere größer ist als der zylindrische Außendurchmesser des Kapillarröhrchens 3, wird das Aufnehmen des Kapillarröhrchens 3 beträchtlich erleichtert.

Wie die Vergrößerung in Fig. 1 jedoch zeigt, ist das Kapillarröhrchen 3 auch bei teilweisem Hineinragen in den Kanalabschnitt 5 noch nicht befestigt. Die endgültige Befestigung des Kapillarröhrchens 3 im Kanalabschnitt 5 des Röhrchenhalters 2 wird durch axiales Drücken des Röhrchenhalters 2 gegen das Kapillarröhrchen 3 vollzogen. Dabei gleitet die konische Innenwandung 23 des Kanalabschnitts 5 entlang des Kapillarröhrchens 3, bis schließlich ein leichter Presssitz erreicht ist. Ein solcher Sitz des Kapillarröhrchens 3 ist beispielsweise in Figur 2 zu erkennen. Das Kapillarröhrchen 3 stößt auch im Presssitz nicht notwendigerweise an die Verengung 20 zwischen Kanalabschnitt 5 und Verbindungsabschnitt 6 an.

Zum Greifen und zum Aufbringen des Gegendrucks beim Entleeren des Kapillarröhrchens 3 dient ein einstückig außen an den Verbindungsabschnitt 6 angeformter, starrer Flansch 25. Dieser ist an dem dem Kapillarröhrchen 3 abgewandten Ende des Verbindungsabschnitts 6 angeordnet. Der Flansch 25 umgibt den Röhrchenhalter 2 vollständig oder partiell.

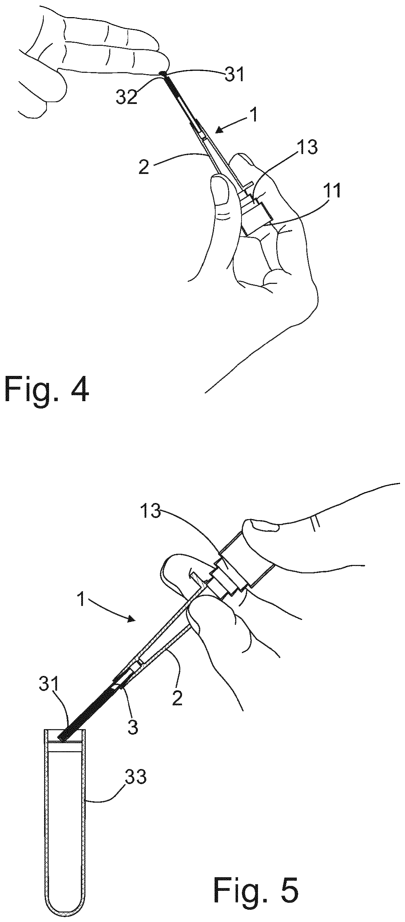

Zur Aufnahme von medizinischen Proben, wie in Figur 4 beispielhaft anhand eines an einem Finger eines Probanden befindlichen Bluttropfens 31 gezeigt, wird das offene Ende 32 des Kapillarröhrchens 3 in Kontakt mit dem Blut(tropfen) 31 bzw. mit der medizinischen Probe gebracht. Das Fluid gelangt ausschließlich durch Kapillarzug in das Kapillarröhrchen 3. Der an dem Röhrchenhalter 2 angeformte Flansch 25 dient hierbei als Greif- und Haltevorrichtung.

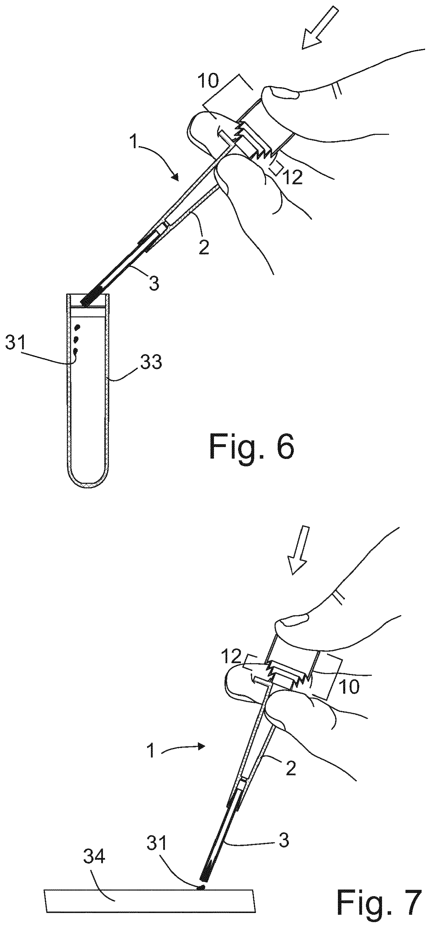

Zum Entleeren des Kapillarröhrchens 3 wird die Pipettiervorrichtung 1, wie Figur 5 zeigt, über einem geeigneten Auffangbehältnis 33 oder über einem Labor-Probenstreifen 34 positioniert. Die Öffnung 11 des radial erweiterten Abschnitts 10 ist beim Entleerungsvorgang von einem Finger oder Daumen bedeckt, und somit verschlossen. Zugleich wird der verformbare Stauchabschnitt 12 durch die Druckkraft des Fingers oder Daumens komprimiert, so dass die Falten zusammengeschoben werden und sich das eingeschlossene Volumen verringert. Der verformbare Abschnitt 12 wird also gestaucht, und so das Fluid aus dem Kapillarröhrchen 3 herausgedrückt. Der Entleerungsvorgang ist beispielhaft in Figur 6 für die Entleerung in einem Auffangbehältnis 33 und in Figur 7 für die Entleerung auf einem Teststreifen 34 dargestellt.

### Bezugszeichenliste

- 1: Pipettiervorrichtung
- 2: Röhrchenhalter
- 3: Kapillarröhrchen
- 4: Aufbewahrungsbehältnis
- 5: Kanalabschnitt
- 6: Verbindungsabschnitt
- 10: radial erweiterter Abschnitt
- 11: Öffnung
- 12: Stauchabschnitt
- 13: Segment
- 14: Mantel
- 15: Innenraum
- 16: Innenraum
- 20: Verengung
- 21: Öffnung
- 22: Fluidstopp
- 23: Innenwandung
- 24: offenes Ende
- 25: Flansch
- 30: Boden
- 31: Blut(tropfen)
- 32: offenes Ende
- 33: Auffangbehältnis
- 34: Teststreifen, Labor-Probenstreifen

## Patentansprüche

1. Pipettiervorrichtung (1) zur Aufnahme und Abgabe von medizinischen Proben, zum Beispiel Körperfluiden, mit einem zylindrischen Kapillarröhrchen (3) und einem Röhrchenhalter (2), wobei der Rörchenhalter (2) besteht aus
- einem Kanalabschnitt (5) zur Aufnahme des Kapillarröhrchens (3),
- einem radial erweiterten Abschnitt (10), der dem Kanalabschnitt (5) abgewandt mit einer Öffnung (11) versehen ist,
- einem Verbindungsabschnitt (6), welcher eine Strömungsverbindung zwischen dem Kanalabschnitt (5) und dem radial erweiterten Abschnitt (10) bildet,
wobei das gemeinsame Volumen des Verbindungsabschnitts (6) und des radial erweiterten Abschnitts (10) ein Mehrfaches des von dem Kapillarröhrchen (3) umschlossenen Kapillarvolumens beträgt und verkleinerbar ist, **dadurch gekennzeichnet, dass** der Röhrchenhalter (2) zur Verkleinerung des gemeinsamen Volumens mit einem in Längsrichtung des Röhrchenhalters (2) verformbar ausgebildeten Stauchabschnitt (12) versehen ist, der einstückig mit dem Verbindungsabschnitt (6) und mit dem radial erweiterten Abschnitt (10) ist, dass der Kanalabschnitt (5) einstückig mit dem Verbindungsabschnitt (6) ausgebildet ist, und dass zwischen dem Kanalabschnitt (5) und dem Verbindungsabschnitt (6) eine Verengung ausgebildet ist, wobei im Bereich der Verengung (20) ein gasdurchlässiger aber für Flüssigkeiten weitgehend undurchlässiger Fluidstopp (22) angeordnet ist.

2. Pipettiervorrichtung nach Anspruch 1, **gekennzeichnet durch** einen starr und einstückig an dem Verbindungsabschnitt (6) angeformten Flansch (25) zum Greifen der Pipettiervorrichtung (1).

3. Pipettiervorrichtung nach einem der vorangehenden Ansprüche, **dadurch gekennzeichnet, dass** der Fluidstopp (22) als sich durch den Kontakt mit Feuchtigkeit verdichtend ausgebildet ist.

4. Pipettiervorrichtung nach einem der vorangehenden Ansprüche, **dadurch gekennzeichnet, dass** die Innenwandung (23) des Kanalabschnittes (5) zu dessen offenem Ende (24) hin konisch erweitert ausgebildet ist.

5. Pipettiervorrichtung nach einem der vorangehenden Ansprüche, **dadurch gekennzeichnet, dass** sich der Querschnitt des Verbindungsabschnittes (6) zu dem Kanalabschnitt (5) hin verjüngt.

6. Pipettiervorrichtung nach einem der vorangehenden Ansprüche, **dadurch gekennzeichnet, dass** der verformbare Abschnitt (12) von Ziehharmonika-förmiger Gestalt ist.

7. Pipettiervorrichtung nach einem der vorangehenden Ansprüche, **dadurch gekennzeichnet, dass** der Röhrchenhalter (2) samt Kanalabschnitt (5), Verbindungsabschnitt (6), dem radial erweiterten Abschnitt (10) und dem Stauchabschnitt (12) ein Spritzgussteil aus polyolefinischem Material, vorzugsweise aus Polyethylen, ist.

8. Pipettiervorrichtung nach einem der vorangehenden Ansprüche, **dadurch gekennzeichnet, dass** das Kapillarröhrchen (3) aus transparentem Polyvinylchlorid oder Polyethylenterephthalat besteht.

## Claims

1. Pipetting device (1) for receiving and dispensing medical samples, for example bodily fluids, having a cylindrical capillary tube (3) and a tube holder (2), wherein the tube holder (2) is composed of
- a channel portion (5) for receiving the capillary tube (3),
- a radially widened portion (10), which is provided with an opening (11) directed away from the channel portion (5),
- a connecting portion (6), which forms a flow connection between the channel portion (5) and the radially widened portion (10),
wherein the common volume of the connecting portion (6) and of the radially widened portion (10) is a multiple of the capillary volume enclosed by the capillary tube (3) and can be reduced, **characterized in that**, in order to reduce the common volume, the tube holder (2) is provided with a compression portion (12) which is deformable in the longitudinal direction of the tube holder (2) and which is integral with the connecting portion (6) and with the radially widened portion (10), **in that** the channel portion (5) is formed integrally with the connecting portion (6), and **in that** a constriction is formed between the channel portion (5) and the connecting portion (6), wherein a fluid stop (22) that is permeable to gas but largely impermeable to liquids is arranged in the region of the constriction (20).

2. Pipetting device according to Claim 1, **characterized by** a flange (25) formed rigidly and integrally on the connecting portion (6) and serving for gripping the pipetting device (1).

3. Pipetting device according to one of the preceding claims, **characterized in that** the fluid stop (22) is designed compacting through contact with moisture.

4. Pipetting device according to one of the preceding claims, **characterized in that** the inner wall (23) of the channel portion (5) is designed widening conically towards its open end.

5. Pipetting device according to one of the preceding claims, **characterized in that** the cross section of the connecting portion (6) tapers towards the channel portion (5).

6. Pipetting device according to one of the preceding claims, **characterized in that** the deformable portion (12) has an accordion-shaped configuration.

7. Pipetting device according to one of the preceding claims, **characterized in that** the tube holder (2), with channel portion (5), connecting portion (6), radially widened portion (10) and compression portion (12), is an injection-moulded part made of polyolefinic material, preferably of polyethylene.

8. Pipetting device according to one of the preceding claims, **characterized in that** the capillary tube (3) is made of transparent polyvinyl chloride or polyethylene terephthalate.

## Revendications

1. Dispositif de pipetage (1) pour la réception et le déchargement d'échantillons médicaux, par exemple de fluides corporels, muni d'un tube capillaire cylindrique (3) et d'un porte-tube (2), le porte-tube (2) étant constitué par :
- une section de canal (5) pour la réception du tube capillaire (3),
- une section élargie radialement (10), qui est munie d'une ouverture (11) détournée de la section de canal (5),
- une section de liaison (6), qui forme une liaison fluidique entre la section de canal (5) et la section élargie radialement (10),
le volume commun de la section de liaison (6) et de la section élargie radialement (10) étant un multiple du volume capillaire entouré par le tube capillaire (3) et pouvant être réduit, **caractérisé en ce que** le porte-tube (2) est muni d'une section de compression (12) configurée sous forme déformable dans la direction longitudinale du porte-tube (2) pour la réduction du volume commun, qui est d'un seul tenant avec la section de liaison (6) et avec la section élargie radialement (10), **en ce que** la section de canal (5) est configurée d'un seul tenant avec la section de liaison (6), et **en ce qu'**un rétrécissement est formé entre la section de canal (5) et la section de liaison (6), un arrêt de fluide (22) perméable aux gaz, mais essentiellement imperméable aux liquides étant agencé dans la zone du rétrécissement (20).

2. Dispositif de pipetage selon la revendication 1, **caractérisé par** une bride (25) rigide et formée d'un seul tenant sur la section de liaison (6) pour la prise du dispositif de pipetage (1).

3. Dispositif de pipetage selon l'une quelconque des revendications précédentes, **caractérisé en ce que** l'arrêt de fluide (22) est configuré sous une forme se compactant par le contact avec de l'humidité.

4. Dispositif de pipetage selon l'une quelconque des revendications précédentes, **caractérisé en ce que** la paroi intérieure (23) de la section de canal (5) est configurée sous forme élargie coniquement vers son extrémité ouverte (24).

5. Dispositif de pipetage selon l'une quelconque des revendications précédentes, **caractérisé en ce que** la section transversale de la section de liaison (6) s'effile vers la section de canal (5).

6. Dispositif de pipetage selon l'une quelconque des revendications précédentes, **caractérisé en ce que** la section déformable (12) est de structure en forme d'accordéon.

7. Dispositif de pipetage selon l'une quelconque des revendications précédentes, **caractérisé en ce que** le porte-tube (2), y compris la section de canal (5), la section de liaison (6), la section élargie radialement (10) et la section de compression (12), est une pièce moulée par injection en matériau polyoléfinique, de préférence en polyéthylène.

8. Dispositif de pipetage selon l'une quelconque des revendications précédentes, **caractérisé en ce que** le tube capillaire (3) est constitué de polychlorure de vinyle ou polyéthylène téréphtalate transparent.
